(19) Europäisches Patentamt
European Patent Office
Office européen des brevets

(11) Veröffentlichungsnummer: **0 002 200**
**B1**

(12) # EUROPÄISCHE PATENTSCHRIFT

(45) Veröffentlichungstag der Patentschrift: **02.09.81**

(51) Int. Cl.³ **C 07 D 239/34**, A 01 N 43/50, C 07 D 239/70, C 07 D 239/90

(21) Anmeldenummer: **78101349.5**

(22) Anmeldetag: **11.11.78**

(54) **N,N-Dialkyl-O-pyrimidinyl-carbaminsäureester, Verfahren zu ihrer Herstellung und ihre Verwendung als Insektizide.**

(30) Priorität: **25.11.77 DE 2752613**

(43) Veröffentlichungstag der Anmeldung: **13.06.79 Patentblatt 79/12**

(45) Bekanntmachung des Hinweises auf die Patenterteilung: **02.09.81 Patentblatt 81/35**

(84) Benannte Vertragsstaaten: **BE CH DE FR GB NL**

(56) Entgegenhaltungen: **FR-A-1 080 289**

(73) Patentinhaber: **BAYER AG, Zentralbereich Patente, Marken und Lizenzen, D-5090 Leverkusen 1, Bayerwerk (DE)**

(72) Erfinder: **Maurer, Fritz, Dr., Roeberstrasse 8, D-5600 Wuppertal 1 (DE)**
Erfinder: **Schröder, Rolf, Dr., Pahlkestrasse 17, D-5600 Wuppertal 1 (DE)**
Erfinder: **Hammann, Ingeborg, Dr., Belfortstrasse 9, D-5000 Köln (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt wenn die Einspruchsgebühr entrichtet worden ist (Art. 99(1) Europäisches Patentübereinkommen).

EP 0 002 200 B1

BUNDESDRUCKEREI BERLIN

N,N-Dialkyl-O-pyrimidinyl-carbaminsäureester, Verfahren zu ihrer Herstellung und ihre Verwendung als Insektizide

Die Erfindung betrifft N,N-Dialkyl-O-pyrimidinyl-carbaminsäureester, mehrere Verfahren zu ihrer Herstellung sowie ihre Verwendung als Insektizide.

Es ist bereits bekanntgeworden, daß N,N-Dialkyl-O-[2-alkylpyrimidin(4)yl]-carbaminsäureester, wie beispielsweise N,N-Dimethyl-O-[2-isopropyl-6-methyl-pyrimidin(4)yl]-carbaminsäureester, insektizide Eigenschaften besitzen (vergleiche US-Patentschrift 26 94 712).

Es wurden die erfindungsgemäßen N,N-Dialkyl-O-pyrimidinyl-carbaminsäureester der Formel I

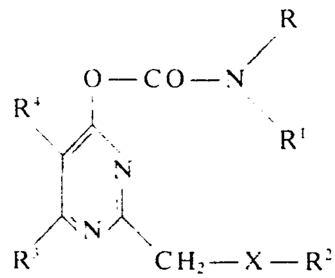

I

in welcher

R, $R^1$ und $R^2$ für gleiches oder verschiedenes $C_{1-6}$-Alkyl stehen,
X für Sauerstoff oder Schwefel steht,
$R^3$ für Wasserstoff oder $C_{1-8}$-Alkyl steht,
$R^4$ für Wasserstoff, $C_{1-6}$-Alkyl oder Halogen steht,

beide Reste $R^3$ und $R^4$ können auch gemeinsam eine $C_3-C_5$-Alkylenbrücke bilden, gefunden.

Diese Verbindungen weisen starke insektizide Eigenschaften auf.

Weiterhin wurde gefunden, daß man die N,N-Dialkyl-O-pyrimidin-carbaminsäureester der Formel I erhält, wenn man

a) N,N-Dialkylcarbaminsäurehalogenide der Formel II

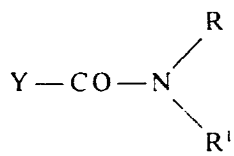

(II)

in welcher

R und $R^1$ die oben angegebene Bedeutung haben und
Y für Halogen, insbesondere für Chlor, steht,

mit substituierten 4-Hydroxy-pyrimidinen der Formel III

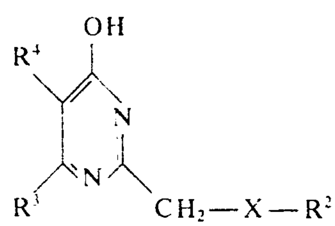

(III)

in welcher

$R^2$, $R^3$ und $R^4$ sowie X die oben angegebene Bedeutung haben,

gegebenenfalls in Gegenwart eines Säureakzeptors oder gegebenenfalls in Gegenwart eine Verdünnungsmittels umsetzt, oder

b) Phosgen mit substituierten 4-Hydroxy-pyrimidinen der Formel III und mit Dialkylaminen der Formel IV

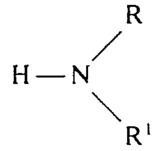

(IV)

in welcher

R und $R^1$ die oben angegebene Bedeutung haben,

gegebenenfalls in Gegenwart eines Säureakzeptors oder gegebenenfalls in Gegenwart eines Verdünnungsmittels umsetzt.

Überraschenderweise zeigen die erfindungsgemäßen N,N-Dialkyl-O-pyrimidinyl-carbaminsäureester eine erheblich höhere insektizide Wirkung als die aus dem Stand der Technik bekannten N,N-Dialkyl-O-[2-alkyl-pyrimidin(4)yl]-carbaminsäureester. Die erfindungsgemäßen Stoffe stellen somit eine Bereicherung der Technik dar.

Verwendet man beispielsweise 2-Methoxymethyl-4-hydroxypyrimidin und nach der Verfahrensvariante a) N,N-Dimethylcarbaminsäurechlorid bzw. nach der Variante b) Phosgen und Dimethylamin als Ausgangsverbindungen, so kann der Reaktionsablauf durch folgendes Formelschema wiedergegeben werden:

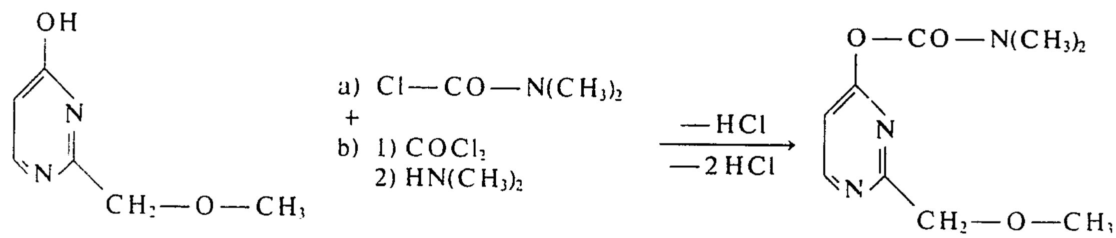


Die als Ausgangsstoffe verwendeten substituierten 4-Hydroxypyrimidine und N,N-Dialkylcarbaminsäurederivate bzw. Dialkylamine sind durch die Formeln II, III und IV allgemein definiert. Vorzugsweise stehen darin jedoch

R, $R^1$ und $R^2$ für geradkettiges oder verzweigtes Alkyl mit 1 bis 4 Kohlenstoffatomen,
$R^3$ für Wasserstoff, geradkettiges oder verzweigtes Alkyl mit 1 bis 5 Kohlenstoffatomen,
$R^4$ für Wasserstoff, geradkettiges oder verzweigtes Alkyl mit 1 bis 4 Kohlenstoffatomen sowie für Chlor oder Brom, oder $R^3$ und $R^4$ bilden gemeinsam eine Alkylengruppe mit 3 bis 5 Kohlenstoffatomen.

Die als Ausgangsstoffe verwendbaren 4-Hydroxypyrimidine der Formel III können nach bekannten Verfahren hergestellt werden: für den Fall, daß X für Sauerstoff steht, aus 2-Chlor-methyl-4-hydroxypyrimidinen und Alkalialkoholaten; für den Fall, daß X für Schwefel steht, aus 2-Chlormethyl-4-hydroxy-pyrimidinen und Alkalisalzen von Thiolen.

Als Beispiele für die als Ausgangsverbindungen verwendbaren 4-Hydroxy-pyrimidine der Formel III seien genannt:

2-Methoxymethyl-4-hydroxy-,
2-Methoxymethyl-4-hydroxy-5-methyl-,
2-Methoxymethyl-4-hydroxy-6-methyl-,
2-Methoxymethyl-4-hydroxy-5,6-dimethyl-,
2-Methoxymethyl-4-hydroxy-5-methyl-6-äthyl-,
2-Methoxymethyl-4-hydroxy-5-äthyl-6-methyl-,
2-Methoxymethyl-4-hydroxy-5-methyl-6-n-propyl-,
2-Methoxymethyl-4-hydroxy-5-n-propyl-6-methyl-,
2-Methoxymethyl-4-hydroxy-5-methyl-6-isopropyl-,
2-Methoxymethyl-4-hydroxy-5-isopropyl-6-methyl-,
2-Methoxymethyl-4-hydroxy-5-methyl-6-n-butyl-,
2-Methoxymethyl-4-hydroxy-5-n-butyl-6-methyl-,
2-Methoxymethyl-4-hydroxy-5-methyl-6-tert.-butyl-,
2-Methoxymethyl-4-hydroxy-5-tert.-butyl-6-methyl-,

2-Methoxymethyl-4-hydroxy-5-chloro-,
2-Methoxymethyl-4-hydroxy-5-bromo-,
2-Methoxymethyl-4-hydroxy-5-chloro-6-methyl-,
2-Methoxymethyl-4-hydroxy-5-bromo-6-methyl-,
2-Äthoxymethyl-4-hydroxy-,
2-Äthoxymethyl-4-hydroxy-5-methyl-,
2-Äthoxymethyl-4-hydroxy-6-methyl-,
2-Äthoxymethyl-4-hydroxy-5,6-dimethyl-,
2-Äthoxymethyl-4-hydroxy-5-methyl-6-äthyl-,
2-Äthoxymethyl-4-hydroxy-5-äthyl-6-methyl-,
2-Isopropoxymethyl-4-hydroxy-,
2-Isopropoxymethyl-4-hydroxy-5-methyl-,
2-Isopropoxymethyl-4-hydroxy-6-methyl-,
2-Isopropoxymethyl-4-hydroxy-5,6-dimethyl-,
2-Isopropoxymethyl-4-hydroxy-5-chloro-6-methyl-,
2-Isopropoxymethyl-4-hydroxy-5-bromo-6-methyl-,
2-Methylthiomethyl-4-hydroxy-,
2-Methylthiomethyl-4-hydroxy-5-methyl-,
2-Methylthiomethyl-4-hydroxy-6-methyl-,
2-Methylthiomethyl-4-hydroxy-5,6-dimethyl-,
2-Methylthiomethyl-4-hydroxy-5-methyl-6-äthyl-,
2-Methylthiomethyl-4-hydroxy-5-äthyl-6-methyl-,
2-Methylthiomethyl-4-hydroxy-5-methyl-6-isopropyl-,
2-Methylthiomethyl-4-hydroxy-6-methyl-5-isopropyl-,
2-Methylthiomethyl-4-hydroxy-5-chloro-6-methyl-,
2-Methylthiomethyl-4-hydroxy-5-bromo-6-methyl-,
2-Methylthiomethyl-4-hydroxy-5-methyl-6-tert.-butyl-,
2-Methylthiomethyl-4-hydroxy-5-tert.-butyl-6-methyl-,
2-Methylthiomethyl-4-hydroxy-6-tert.-butyl-,
2-Äthylthiomethyl-4-hydroxy-,
2-Äthylthiomethyl-4-hydroxy-5-methyl-,
2-Äthylthiomethyl-4-hydroxy-6-methyl-,
2-Äthylthiomethyl-4-hydroxy-5,6-dimethyl-,
2-Isopropylthiomethyl-4-hydroxy-,
2-Isopropylthiomethyl-4-hydroxy-5-methyl-,
2-Isopropylthiomethyl-4-hydroxy-6-methyl-,
2-Isopropylthiomethyl-4-hydroxy-5,6-dimethyl-pyrimidin,
2-Methylthiomethyl-4-hydroxy-1,3-diaza-bicyclo(4,3,0)-nona-2,4,6-trien und
2-Äthylthiomethyl-4-hydroxy-1,3-diaza-bicyclo-(4,3,0)-nona-2,4,6-trien.

Als Beispiele für die nach Variante a) einzusetzenden Carbaminsäurehalogenide der Formel II seien Dimethyl-carbaminsäurechlorid und Diäthyl-carbaminsäurechlorid genannt.

Als Verdünnungsmittel kommen alle inerten organischen Lösungsmittel in Frage. Als solche seien beispielsweise Kohlenwasserstoffe, wie Benzol, Toluol, Xylol, chlorierte Kohlenwasserstoffe, wie Di-, Tri- oder Tetrachlormethan sowie Chlorbenzol oder o-Dichlorbenzol, Äther wie Diäthyl- und Dibutyläther, Ketone, wie Aceton, Methyläthyl-, Methyl-isopropyl- und Methyl-isobutylketon sowie Nitrile, wie Acetonitril und Propionitril genannt.

Als Säureakzeptoren können bei allen Varianten die üblichen Säurebindemittel Verwendung finden Als solche seien beispielsweise Alkalicarbonate und -alkoholate, wie Natrium- und Kaliumcarbonat, Natrium- und Kaliummethylat bzw. -äthylat, ferner aliphatische, aromatische oder heterocyclische Amine, beispielsweise Triäthylamin, Dimethylanilin, Dimethylbenzylamin und Pyridin genannt.

Die Reaktionstemperatur kann bei allen Verfahrensvarianten innerhalb eines größeren Bereichs variiert werden. Im allgemeinen arbeitet man zwischen 0 und 150° C, vorzugsweise bei 20 bis 100° C.

Die Umsetzung wird bei sämtlichen Varianten im allgemeinen bei Normaldruck durchgeführt.

Zur Durchführung des erfindungsgemäßen Verfahrens werden bei Variante a) die Dialkylcarbaminsäurechloride vorzugsweise in einem Überschuß von 10 bis 30 Molprozent, bezogen auf die 4-Hydroxy-pyrimidine eingesetzt. Die Reaktionspartner werden im allgemeinen in einem organischen Verdünnungsmittel dispergiert, mehrere Stunden unter Rückfluß zum Sieden erhitzt und anschließend das Verdünnungsmittel abdestilliert. Bei der Variante b) des erfindungsgemäßen Verfahrens werden aus Hydroxypyrimidinen und Phosgen vorzugsweise im Molverhältnis 1 : 1 und in Gegenwart eines der o. g. Säureakzeptoren, die entsprechenden Chlorameisensäureester hergestellt und diese Produkte bevorzugt in situ mit sekundären Aminen, entweder im Molverhältnis 1 : 1 in Gegenwart eines der oben genannten Säureakzeptoren oder mit überschüssigen sekundären Aminen umgesetzt. Die einzelnen Verfahrensschritte werden im allgemeinen in organischen Verdünnungsmitteln durchgeführt, welche nach Reaktionsende abdestilliert werden.

Die erfindungsgemäßen Verbindungen fallen oft in Form von Ölen an, die sich zum Teil nicht unzersetzt destillieren lassen, jedoch durch sogenanntes »Andestillieren«, d. h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechnungsindex. Einige Verbindungen fallen in kristalliner Form an und werden durch ihren Schmelzpunkt charakterisiert.

Die Wirkstoffe eignen sich bei guter Pflanzenverträglichkeit und günstiger Warmblütertoxizität zur Bekämpfung von tierischen Schädlingen, insbesondere Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadillidium vulgare, Porcellio scaber.
Aus der Ordnung der Diplopoda z. B. Blaniulus guttulatus.
Aus der Ordnung der Chilopoda z. B. Geophilus carpophagus, Scutigera spec.
Aus der Ordnung der Symphyla z. B. Scutigerella immaculata.
Aus der Ordnung der Thysanura z. B. Lepisma saccharina.
Aus der Ordnung der Collembola z. B. Onychiurus armatus.
Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z. B. Forficula auricularia.
Aus der Ordnung der Isoptera z. B. Reticulitermes spp..
Aus der Ordnung der Anoplura z. B. Phylloxera vastatrix, Pemphigus spp., Pediculus humanus corporis, Haematopinus spp., Linognathus spp.
Aus der Ordnung der Mallophaga z. B. Trichodectes spp., Damalinea spp.
Aus der Ordnung der Thysanoptera z. B. Hercinothrips femoralis, Thrips tabaci.
Aus der Ordnung der Heteroptera z. B. Eurygaster spp., Dysdercus intermedius, Piesma quadrata, Cimex lectularius, Rhodnius prolixus, Triatoma spp.
Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp..
Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z. B. Diprion spp., Hoplocampa spp., Lasius spp., Monomorium pharaonis, Vespa spp.
Aus der Ordnung der Diptera z. B. Aëdes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z. B. Xenopsylla cheopis, Ceratophyllus spp..

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für

Saatgut, ferner in Formulierungen mit Brennsätzen, wie Räucherpatronen, -dosen, -spiralen u. ä. sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen infrage Aromate, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z. B. Alkylaryl-polyglykol-äther, Alkylsulfonate, Alkylsulfate, Arylsulfonate sowie Eiweißhydrolysate; als Dispergiermittel: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekalkten Unterlagen aus.

## Beispiel A

### Doralis-Test (systemische Wirkung)

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia faba), die stark von der schwarzen Bohnenlaus (Doralis fabae) befallen sind, angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach der gewünschten Zeit wird die Abtötung in % bestimmt. Dabei bedeutet 100%, daß alle Bohnenläuse abgetötet wurden; 0% bedeutet, daß keine Bohnenläuse abgetötet wurden.

Bei diesem Test zeigen z. B. die folgenden Verbindungen der Herstellungsbeispiele überlegene Wirksamkeit gegenüber dem Stand der Technik: 1, 10, 9, 7, 8, 5, 3, 11, 12.

## Herstellungsbeispiele

### Beispiel 1

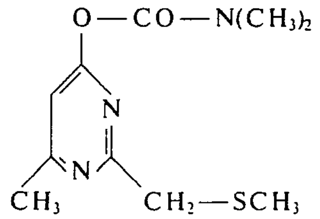

Ein Gemisch aus 17 g (0,1 Mol) 2-Methylthiomethyl-4-hydroxy-6-methylpyrimidin, 20,7 g (0,15 Mol) Kaliumcarbonat, 200 ml Acetonitril und 11,8 g (0,11 Mol) N,N-Dimethylcarbamidsäurechlorid wird 12 Stunden unter Rückfluß gekocht. Nach Abkühlen auf Raumtemperatur filtriert man das Reaktionsgemisch und dampft dann das Filtrat im Vakuum ein. Zurück bleiben 18 g (75% der Theorie) N,N-Dimethyl-O-[2-methylthiomethyl-6-methyl-pyrimidin(4)yl]-carbaminsäureester in Form eines braunen Öles mit dem Brechungsindex $n_D^{21}$ : 1,5541.

Analog Beispiel 1 können die folgenden Verbindungen der Formel I

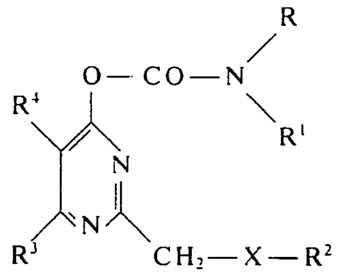

(I)

hergestellt werden:

| Beispiel Nr. | R | $R^1$ | $R^2$ | $R^3$ | $R^4$ | X | Ausbeute (% der Theorie) | Physikal. Daten (Brechungsindex, Schmelzpunkt °C) |
|---|---|---|---|---|---|---|---|---|
| 2 | $CH_3$ | $CH_3$ | $CH_3$ | $CH_3$ | H | O | 84 | $n_D^{24}$: 1,5048 |
| 3 | $CH_3$ | $CH_3$ | $CH_3$ | $CH_3$ | Br | O | 46 | 76 |
| 4 | $CH_3$ | $CH_3$ | $C_3H_7$-iso | $CH_3$ | H | O | 83 | $n_D^{24}$: 1,4998 |
| 5 | $CH_3$ | $CH_3$ | $CH_3$ | $CH_3$ | Cl | O | 42 | 57 |
| 6 | $CH_3$ | $CH_3$ | $C_3H_7$-n | $CH_3$ | H | S | 93 | $n_D^{22}$: 1,5756 |
| 7 | $CH_3$ | $CH_3$ | $CH_3$ | $CH_3$ | Br | S | 69 | $n_D^{22}$: 1,5305 |
| 8 | $CH_3$ | $CH_3$ | $CH_3$ | $CH_3$ | $CH_3$ | O | 71 | 45 |
| 9 | $CH_3$ | $CH_3$ | $CH_3$ | $CH_3$ | Cl | S | 73 | $n_D^{23}$: 1,5585 |
| 10 | $CH_3$ | $CH_3$ | $CH_3$ | $CH_3$ | $CH_3$ | S | 75 | 47 |
| 11 | $CH_3$ | $CH_3$ | $C_2H_5$ | $CH_3$ | H | S | 76 | $n_D^{20}$: 1,5042 |
| 12 | $CH_3$ | $CH_3$ | $C_2H_5$ | $CH_3$ | $CH_3$ | S | 89 | $n_D^{20}$: 1,4957 |
| 13 | $CH_3$ | $CH_3$ | $C_2H_5$ | $CH_3$ | H | O | 72 | $n_D^{20}$: 1,5245 |
| 14 | $CH_3$ | $CH_3$ | $CH_3$ | H | H | S | | |
| 15 | $C_2H_5$ | $C_2H_5$ | $CH_3$ | $CH_3$ | $CH_3$ | S | | |
| 16 | $CH_3$ | $CH_3$ | $CH_3$ | $CH_3$ | $C_2H_5$ | S | | |
| 17 | $CH_3$ | $CH_3$ | $CH_3$ | $CH_3$ | $C_3H_7$-iso | S | | |

| Beispiel Nr. | R | $R^1$ | $R^2$ | $R^3$ | $R^4$ | X | Ausbeute (% der Theorie) | Schmelzpunkt °C |
|---|---|---|---|---|---|---|---|---|
| 18 | $CH_3$ | $CH_3$ | $CH_3$ | $C_4H_9$-tert. | H | S | | |
| 19 | $CH_3$ | $CH_3$ | $CH_3$ | $C_4H_9$-tert. | $CH_3$ | S | | |
| 20 | $CH_3$ | $CH_3$ | $CH_3$ | H | $CH_3$ | S | | |
| 21 | $CH_3$ | $CH_3$ | $CH_3$ | $CH_2-CH_2-CH_2$ | | S | 75 | 63 |
| 22 | $CH_3$ | $CH_3$ | $C_2H_5$ | $CH_2-CH_2-CH_2$ | | S | | |
| 23 | $CH_3$ | $CH_3$ | $C_3H_7$-n | $CH_3$ | $CH_3$ | S | | |
| 24 | $CH_3$ | $CH_3$ | $C_3H_7$-iso | $CH_3$ | $CH_3$ | S | | |
| 25 | $CH_3$ | $CH_3$ | $CH_3$ | $CH_3$ | $C_4H_9$-n | S | | |

Die als Ausgangsmaterialien zu verwendenden 4-Hydroxypyrimidine können z. B. wie folgt hergestellt werden:

Beispiel a

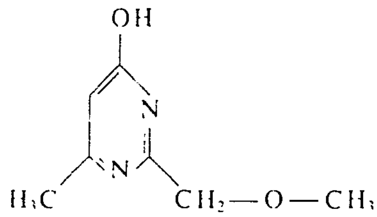

Zu einer Lösung von 80 g (0,5 Mol) 2-Chlormethyl-4-hydroxy-6-methylpyrimidin [Darstellung s. T. Kato, H. Yamanaka und J. Kawamata Yakugaku Zasshi 89, 460—463 (1969); Chemical Abstracts 71, 70653a (1969)] in 100 ml Methanol tropft man eine Lösung von 60 g (1,1 Mol) Natriummethylat in 200 ml Methanol. Das Gemisch wird 2 Stunden bei 55—60° C gerührt, dann auf Raumtemperatur abgekühlt und mit wäßriger Salzsäure auf pH 5—6 gebracht. Nach Abdestillieren des Lösungsmittels trocknet man den Rückstand bei 70° C, verreibt ihn mit 800 ml warmem Aceton, filtriert vom Ungelösten und dampf das Filtrat im Vakuum zur Trockne ein. Zurück bleiben 60 g (78% der Theorie) 2-Methoxymethyl-4-hydroxy-6-methylpyrimidin in Form farbloser Kristalle mit dem Schmelzpunkt 101° C.

Beispiel b

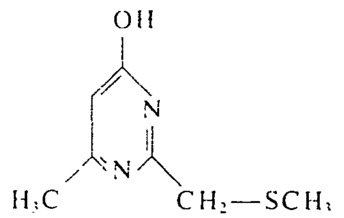

Ein Gemisch aus 119 g (0,75 Mol) 2-Chlormethyl-4-hydroxy-6-methylpyrimidin, 52,5 g (0,75 Mol) Natriummethylmercaptid und 700 ml Acetonitril wird 3 Stunden unter Rückfluß gekocht. Dann filtriert man das heiße Reaktionsgemisch und dampft das Filtrat im Vakuum zur Trockne ein. Man erhält so 68 g (53% der Theorie) 2-Methylthiomethyl-4-hydroxy-6-methylpyrimidin in Form eines beigen Pulvers mit dem Schmelzpunkt 138° C.

Analog einem der Beispiele a oder b können die folgenden Verbindungen der Formel III

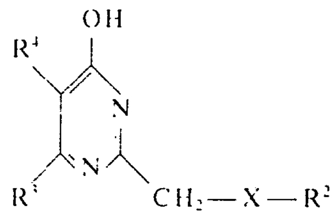

(III)

hergestellt werden:

| Beispiel | $R^2$ | $R^3$ | $R^4$ | X | Ausbeute (% der Theorie) | Schmelzpunkt °C |
|---|---|---|---|---|---|---|
| c | $C_3H_7$-iso | $CH_3$ | H | O | 67 | 61 |
| d | $C_3H_7$-n | $CH_3$ | H | S | 86 | 87 |
| e | $CH_3$ | $CH_3$ | Cl | O | 88 | 142 |
| f | $C_3H_7$-iso | $CH_3$ | Cl | O | 51 | 206(Z) |
| g | $CH_3$ | $CH_3$ | Br | O | 97 | 148 |
| h | $CH_3$ | $CH_3$ | Cl | S | 56 | 165(Z) |
| i | $CH_3$ | $CH_3$ | $CH_3$ | S | 33 | 142 |
| j | $CH_3$ | $CH_3$ | Br | S | 58 | 137 |
| k | $CH_3$ | $CH_3$ | $CH_3$ | O | 89 | 112 |
| l | $C_2H_5$ | $CH_3$ | H | O | 84 | 68 |
| m | $C_2H_5$ | $CH_3$ | H | S | 56 | 132 |
| n | $C_2H_5$ | $CH_3$ | $CH_3$ | S | 43 | 122 |
| o | $CH_3$ | H | H | S | | |
| p | $CH_3$ | $CH_3$ | $C_2H_5$ | S | | |
| q | $CH_3$ | $CH_3$ | $C_3H_7$-iso | S | | |
| r | $CH_3$ | $C_4H_9$-tert. | H | S | | |
| s | $CH_3$ | $C_4H_9$-tert. | $CH_3$ | S | | |
| t | $CH_3$ | H | $CH_3$ | S | | |
| u | $CH_3$ | $CH_2-CH_2-CH_2$ | | S | 38 | 170 |
| v | $C_2H_5$ | $CH_2-CH_2-CH_2$ | | S | | |
| w | $C_3H_7$-n | $CH_3$ | $CH_3$ | S | | |
| x | $C_3H_7$-iso | $CH_3$ | $CH_3$ | S | | |
| y | $CH_3$ | $CH_3$ | $C_4H_9$-n | S | | |

**Patentansprüche**

1. N,N-Dialkyl-O-pyrimidinyl-carbaminsäureester der Formel I

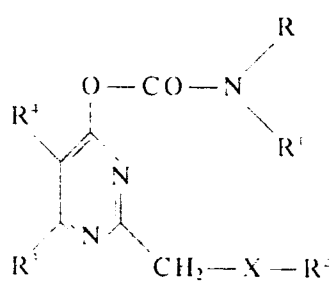

(I)

in welcher

R, $R^1$ und $R^2$ für gleiches oder verschiedenes $C_{1-6}$-Alkyl stehen,
X für Sauerstoff oder Schwefel steht,
$R^3$ für Wasserstoff oder $C_{1-8}$-Alkyl steht,
$R^4$ für Wasserstoff, $C_{1-6}$-Alkyl oder Halogen steht,

beide Reste $R^3$ und $R^4$ können auch gemeinsam eine $C_3-C_5$-Alkylenbrücke bilden.

2. Verfahren zur Herstellung der N,N-Dialkyl-O-pyrimidinylcarbaminsäureester der Formel I gemäß Anspruch 1, dadurch gekennzeichnet, daß man

a) N,N-Dialkylcarbaminsäurehalogenide der Formel II

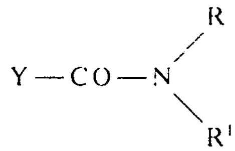

(II)

in welcher

R und $R^1$ die oben angegebene Bedeutung haben und
Y für Halogen, insbesondere für Chlor, steht,

mit substituierten 4-Hydroxy-pyrimidinen der Formel III

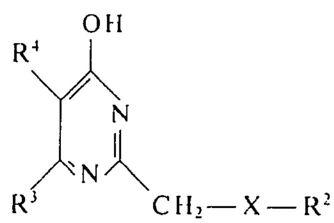

(III)

in welcher

$R^2$, $R^3$ und $R^4$ sowie X die oben angegebene Bedeutung haben,

gegebenenfalls in Gegenwart eines Säureakzeptors oder gegebenenfalls in Gegenwart eines Verdünnungsmittels umsetzt, oder

b) Phosgen mit substituierten 4-Hydroxy-pyrimidinen der Formel III und mit Dialkylaminen der Formel IV

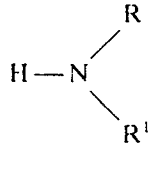

(IV)

in welcher

R und $R^1$ die oben angegebene Bedeutung haben,

gegebenenfalls in Gegenwart eines Säureakzeptors oder gegebenenfalls in Gegenwart eines Verdünnungsmittels umsetzt.

3. Insektizide Mittel gekennzeichnet durch einen Gehalt an N,N-Dialkyl-O-pyrimidinyl-carbaminsäureester gemäß Anspruch 1.

4. Verwendung von N,N-Dialkyl-O-pyrimidinyl-carbaminsäureester gemäß Anspruch 1 zur Bekämpfung von Insekten.

5. Verfahren zur Bekämpfung von Insekten, dadurch gekennzeichnet, daß man N,N-Dialkyl-O-pyrimidinyl-carbaminsäureester gemäß Anspruch 1 auf Insekten und/oder ihren Lebensraum einwirken läßt.

6. Verfahren zur Herstellung insektizider Mittel, dadurch gekennzeichnet, daß man N,N-Dialkyl-O-pyrimidinyl-carbaminsäureester gemäß Anspruch 1 mit Streckmitteln und/oder oberflächenaktiven Stoffen vermischt.

**Claims**

1. N,N-Dialkyl-O-pyrimidinyl-carbamic acid esters of the formula I

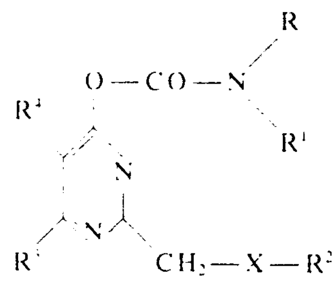

(I)

in which

R, $R^1$ and $R^2$ represent identical or different $C_{1-6}$ alkyl,
X represents oxygen or sulphur,
$R^3$ represents hydrogen or $C_{1-8}$ alkyl,
$R^4$ represents hydrogen, $C_{1-6}$ alkyl or halogen and the two radicals $R^3$ and $R^4$ can also conjointly form a $C_3-C_5$ alkylene bridge.

2. Process für the preparation of the N,N-dialkyl-O-pyrimidinyl-carbamic acid esters of the formula I according to Claim 1, characterised in that

a) N,N-dialkylcarbamic acid halides of the formula II

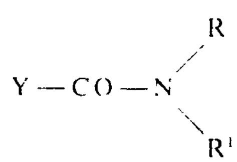

(II)

in which

R and $R^1$ have the abovementioned meaning and
Y represents halogen, especially chlorine,

are reacted with substituted 4-hydroxy-pyrimidines of the formula III

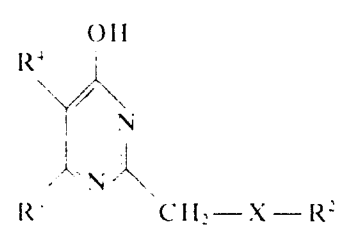

(III)

in which

$R^2$, $R^3$, $R^4$ and X have the abovementioned meaning,

if appropriate in the presence of an acid acceptor or, if appropriate, in the presence of a diluent, or

b) phosgene is reacted with substituted 4-hydroxypyrimidines of the formula (III) and with dialkylamines of the formula IV

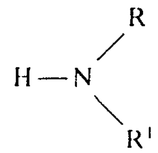

(IV)

in which

R and $R^1$ have the abovementioned meaning,

if appropriate in the presence of an acid acceptor or, if appropriate, in the presence of a diluent.

3. Insecticidal agents characeterised in that they contain N,N-dialkyl-O-pyrimidinyl-carbamic acid esters according to Claim 1.
4. Use of N,N-dialkyl-O-pyrimidinyl-carbamic acid esters according to Claim 1 für combating insects.
5. Process für combating insects characetised in that N,N-dialkyl-O-pyrimidinyl-carbamic acid esters according to Claim 1 are allowed to act on insects and/or their habitat.
6. Process for the preparation of insecticidal agents, characterised in that N,N-dialkyl-O-pyrimidinyl-carbamic acid esters according to Claim 1 are mixed with extenders and/or surface-active agents.

**Revendications**

1. Des esters de O-pyrimidinyle d'acides N,N-dialkylcarbamiques de formule (I):

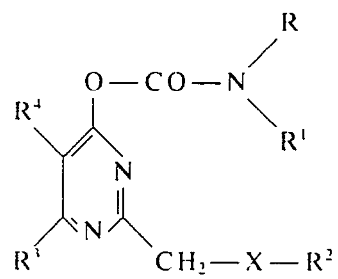

(I)

dans laquelle

R, $R^1$ et $R^2$ représent des groupes alkyle en $C_1$ à $C_6$ égaux ou différents,
X est de l'oxygène ou du soufre
$R^3$ est de l'hydrogène ou un groupe alkyle en $C_1$ à $C_8$
$R^4$ est de l'hydrogène, un groupe alkyle en $C_1$ à $C_6$ ou un halogène,

les deux restes $R^3$ et $R^4$ peuvent aussi former ensemble un pont alkylène en $C_3-C_5$.

2. Procédé de production des esters de O-pyrimidinyle d'acides N,N-dialkylcarbamiques de formule (I) suivant la revendication 1, caractérisé en ce que:

(a) on fait réagir des halogénures d'acides N,N-dialkylcarbamiques de formule (II)

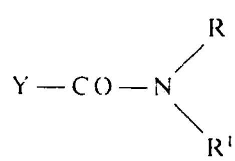

(II)

dans laquelle

R et $R^1$ ont la définition indiquée ci-dessus, et
Y est un halogène, en particulier le chlore,

avec des 4-hydroxypyrimidines substituées de formule (III)

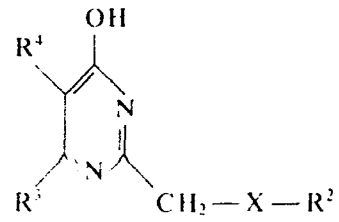

(III)

dans laquelle

$R^2$, $R^3$ et $R^4$ ainsi que X ont la définition indiquée ci-dessus, éventuellement en presence d'un accepteur d'acide, ou le cas échéant, en présence d'un diluant, ou

(b) on fait réagir du phosgène avec des 4-hydroxypyrimidines substituées de formule (III) et avec des dialkylamines de formule (IV)

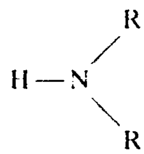

(IV)

dans laquelle

R et $R^1$ ont la définition indiquée ci-dessus, éventuellement en présence d'un accepteur d'acide ou, le cas échéant, en présence d'un diluant.

3. Compositions insecticides, caractérisées par une teneur en esters de O-pyrimidinyle d'acides N,N-dialkylcarbamiques suivant la revendication 1.

4. Utilisation d'esters de O-pyrimidinyle D-acides N,N-dialkylcarbamiques suivant la revendication 1 pour la lutte contre des insectes.

5. Procédé de lutte contre des insectes caractérisé en ce qu'on fait agir des esters de O-pyrimidinyle d'acides N,N-dialkylcarbamiques suivant la revendication 1 sur des insectes et/ou sur leur habitat.

6. Procédé de production de compositions insecticides, caractérisé en ce qu'on melange des esters de O-pyrimidinyle d'acides N,N-dialkylcarbamiques suivant la revendication 1 avec des diluants et/ou des matières tensioactives.